# EUROPEAN PATENT APPLICATION

(11) **EP 2 371 824 A1**
(43) Date of publication of application: **05.10.2011**
(21) Application number: 10155790.8
(22) Date of filing: 08.03.2010
(51) Int. Cl.: C07D 307/80

(54) **Crystalline dronedarone salts**

(71) Applicant: Ratiopharm GmbH, 89079 Ulm (DE)
(72) Inventor: STOHANDL, Jiri, 592 55 Bobrova 236 (CZ); SINDELAROVA, Jana, 798 45 Jednov (CZ)
(74) Representative: Teipel, Stephan

(57) **Abstract**

The present invention relates to crystalline dronedarone salts, a process for their preparation as well as pharmaceutical compositions comprising these salts.

## Description

The present invention relates to crystalline dronedarone salts, a process for their preparation as well as pharmaceutical compositions comprising these salts.

Dronedarone (N-[2-butyl-3-[4-[3-(dibutylamino)propoxy]benzoyl]-5-benzofuranyl]methane-sulfonamide) has the following chemical structure:

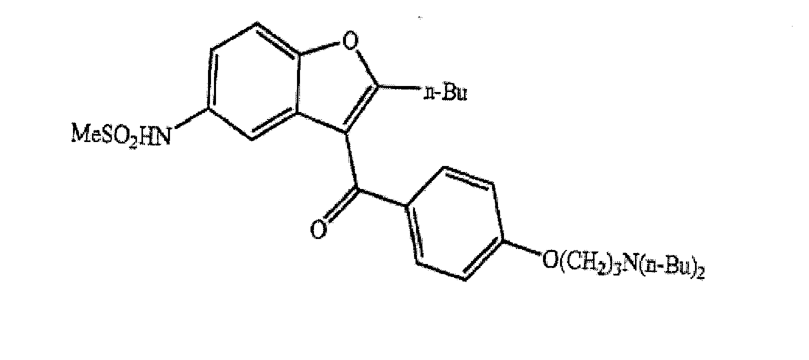

From EP 0 471 609 A2 dronedarone is known as a drug for the treatment of arrhythmia. This document also discloses a method for preparing dronedarone as well as a method for preparing dronedarone hydrochloride. According to this method, first dronedarone free base is prepared and the crude product thus obtained is purified by elution chromatography on a silica column. The purified dronedarone is then dissolved in anhydrous ethyl acetate. A solution of hydrochloric acid in ether is added and the hydrochloride salt is precipitated.

A different method for the preparation of dronedarone hydrochloride is disclosed in US 2004/0048921 A1. According to this method, dronedarone hydrochloride is first obtained in crude from. From this product dronedarone free base is obtained in crude form and finally the dronedarone free base in crude form is converted into dronedarone hydrochloride using isopropanol as solvent.

When repeating the prior art preparation methods, it was surprisingly found that none of these methods led to any crystalline product. Only brown sticky amorphous clot could be isolated. Variation of concentration and temperature did not bring the desired result. Also the use of further different solvents such as diethyl ether and acetone instead of ethyl acetate and isopropanol was unsuccessful. Furthermore, even recrystallization of the sticky gum from other solvents, such as isopropanol, ethyl acetate, ethyl acetate containing water, isopropyl acetate and acetone, was unsuccessful. Only amorphous powders could be obtained. Even trials to obtain different salts like the mesylate, oxalate and fumarate did not lead to the desired crystalline product.

Further investigations revealed that the dronedarone free base purified by elution chromatography on a silica column according to the prior art methods still contains small amounts of impurities, which prevent the dronedarone salts from crystallizing. In was in particular found that dronedarone free base resulting from the synthesis contained oligomeric impurities not detected by HPLC with UV detection that made impossible successful crystallization of any salt. It was surprisingly found that in particular oligomeric impurities which are amines of surfactant character in small or even very little amount inhibit dronedarone from crystallization.

It was now found that this problem can be overcome by further purification of the dronedarone free base before preparing the desired salt. If the dronedarone free base has a very high chemical purity and in particular is substantially free of impurities having a molecular weight being higher than the molecular weight of dronedarone free base, such as oligomeric impurities, salts of dronedarone readily crystallize from their solution. Thus, the present invention provides a salt of dronedarone, which is crystalline.

The salt of the present invention is considered as crystalline if at least 80 % by weight of the total salt is in crystalline form, preferably at least 90 %, more preferably at least 95 %, such as at least 99 %. Most preferably the salt of the present invention is substantially in crystalline form and in particular more than 99.5 % by weight, such as 100 % by weight of the total salt is crystalline. The crystallinity of the salt can be determined, for example, by XRPD.

Preferably, the salt of the present invention has a chemical purity of > 99 %, more preferably of > 99.5 %, such as ≥ 99.7 % and in particular ≥99.8 %. The chemical purity of the salt is determined by HPLC.

In a particularly preferred embodiment the salt of the present invention is substantially free of impurities having a molecular weight being higher than the molecular weight of dronedarone free base, such as oligomers or polymers being obtained as undesired by-products in the preparation of the dronedarone. "Substantially free" means that the salt contains no or as little as possible of the high molecular weight impurities. Preferably, the amount of the high molecular weight impurities is such as obtained when adding diethyl ether to crude dronedarone free base in a weight ratio of dronedarone free base to diethyl ether of about 1:40, stirring for 30 minutes at 23°C, filtering off the precipitated impurities and recovering the dronedarone free base from the solution.

For the preparation of the salt of the present invention any suitable acid, such as an organic or inorganic acid can be used. Preferably, the acid is a pharmaceutically acceptable acid. Most preferred acids are hydrochloric acid, hydrobromic acid, methanesulfonic acid, toluenesulfonic acid, ethanesulfonic acid, sulfuric acid and fumaric acid, resulting preferably in dronedarone hydrochloride, dronedarone mesylate, dronedarone hemisulfate and dronedarone fumarate.

The present invention furthermore relates to new polymorphic forms of dronedarone hydrochloride, dronedarone mesylate, dronedarone hemisulfate and dronedarone fumarate. The polymorphic forms of the crystalline salts are defined by characteristic peaks of their XRPD patterns. Alternatively, the polymorphic forms of the crystalline salts are characterized by their melting points as measured by DSC using a Perkin-Elmer DSC7 apparatus at a scanning rate of 10.0°C/min. As melting point the peak in the DSC is defined.

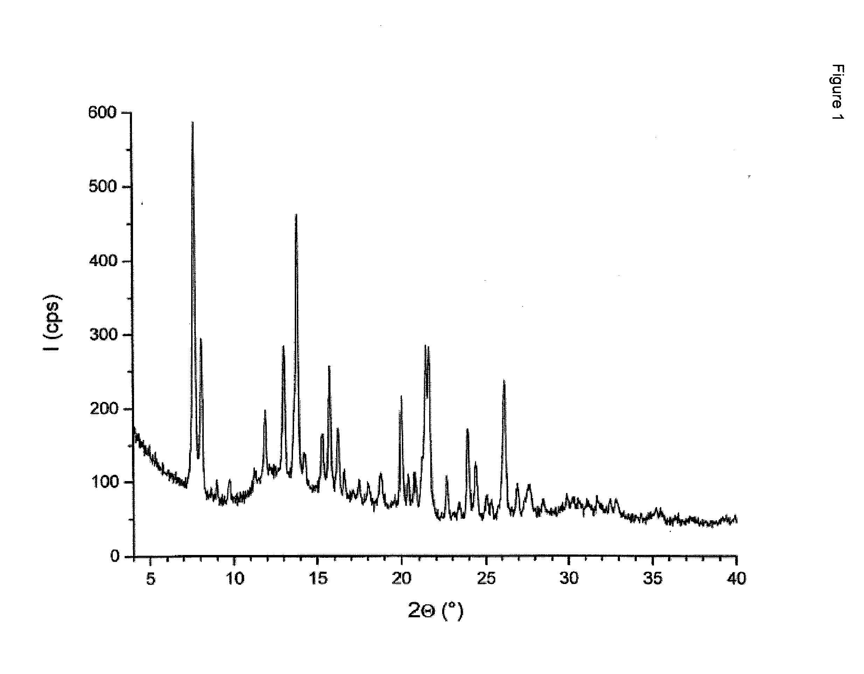

Crystalline dronedarone hydrochloride in the polymorphic form of the present invention shows an XRPD pattern having characteristic peaks at 7.7 ± 0.2, 13.8 ± 0.2, 21.7 ± 0.2 and 26.2 ± 0.2 degree 2-Theta. Preferably, the XRPD pattern has characteristic peaks at 7.7 ± 0.2, 13.0 ± 0.02, 13.8 ± 0.02, 15.7 ± 0.02, 21.4 ± 0.02, 21.7 ± 0.02 and 26.2 ± 0.02 degree 2-Theta. The XRPD pattern of crystalline dronedarone hydrochloride of the present invention is shown in figure 1. The most intense peaks of this XRPD pattern are summarized in the following table 1.

**Table 1**

| **Degree 2-Theta (± 0.2)** | **Relative Intensity** |
|---|---|
| 7.7 | 100 |
| 8.1 | 45 |
| 9.0 | 6 |
| 9.7 | 6 |
| 11.3 | 6 |
| 11.9 | 26 |
| 13.0 | 45 |
| 13.8 | 84 |
| 14.3 | 13 |
| 15.3 | 20 |
| 15.7 | 40 |
| 16.2 | 22 |
| 16.6 | 11 |
| 17.5 | 8 |
| 18.1 | 7 |
| 18.8 | 11 |
| 20.0 | 34 |
| 20.5 | 12 |
| 20.8 | 13 |
| 21.4 | 47 |
| 21.7 | 50 |
| 22.7 | 11 |
| 23.4 | 5 |
| 24.0 | 27 |
| 24.4 | 17 |
| 25.0 | 8 |
| 25.3 | 7 |
| 26.2 | 41 |
| 26.9 | 12 |
| 27.7 | 11 |
| 28.5 | 8 |
| 29.9 | 9 |
| 30.6 | 8 |
| 31.1 | 8 |
| 31.7 | 8 |
| 32.5 | 8 |
| 32.9 | 8 |

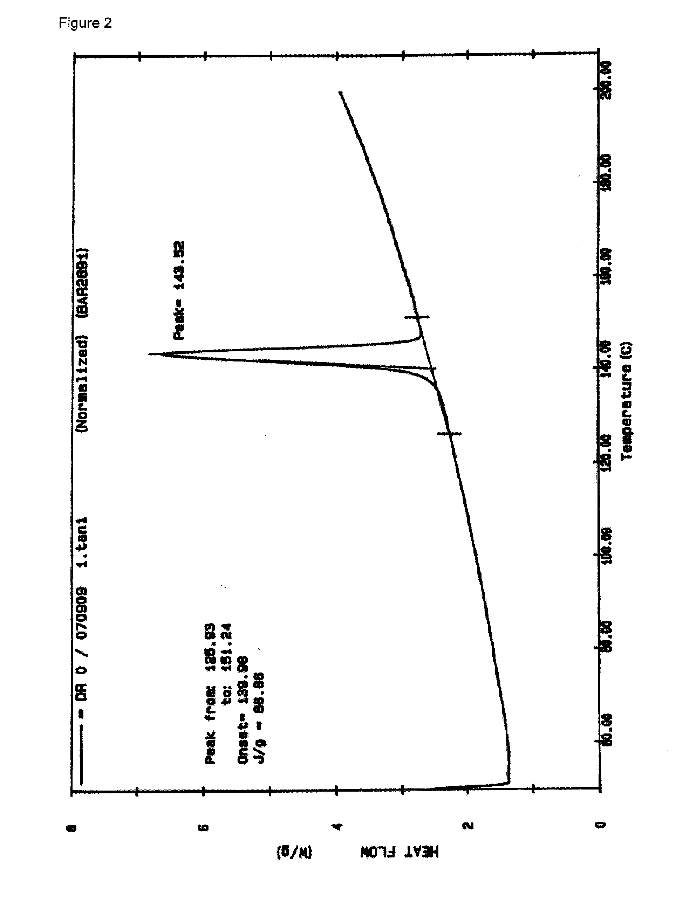

Crystalline dronedarone hydrochloride of the present invention has a melting temperature of 144 ± 1 °C. The DSC of this compound is shown in figure 2.

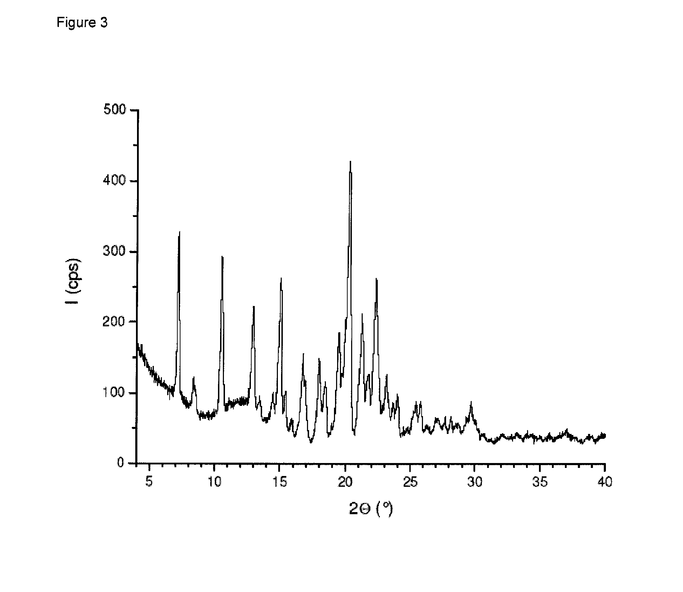

Crystalline dronedarone mesylate in the polymorphic form of the present invention shows an XRPD pattern having characteristic peaks at 7.2 ± 0.2, 10.6 ± 0.2, 20.3 ± 0.2 and 22.3 ± 0.2 degree 2-Theta. Preferably, the XRPD pattern has characteristic peaks at 7.2 ± 0.2, 10.6 ± 0.2, 13.0 ± 0.2, 15.0 ± 0.2, 20.3 ± 0.2, 21.3 ± 0.2 and 22.3 ± 0.2 degree 2-Theta. The XRPD pattern of crystalline dronedarone mesylate of the present invention is shown in figure 3. The most intense peaks of this XRPD pattern are summarized in the following table 2.

**Table 2**

| **Degree 2-Theta (± 0.2)** | **Relative Intensity** |
|---|---|
| 7.2 | 60 |
| 8.4 | 8 |
| 10.6 | 62 |
| 13.0 | 46 |
| 13.5 | 14 |
| 14.5 | 16 |
| 15.0 | 57 |
| 15.5 | 18 |
| 15.9 | 6 |
| 16.7 | 28 |
| 17.0 | 18 |
| 18.0 | 30 |
| 18.5 | 21 |
| 19.5 | 39 |
| 20.3 | 100 |
| 21.3 | 46 |
| 21.7 | 24 |
| 22.3 | 59 |
| 23.1 | 24 |
| 24.0 | 16 |
| 25.2 | 9 |
| 25.5 | 14 |
| 25.8 | 15 |
| 26.9 | 7 |
| 27.2 | 8 |
| 27.7 | 8 |
| 28.1 | 8 |
| 28.5 | 6 |
| 28.8 | 6 |
| 29.7 | 15 |

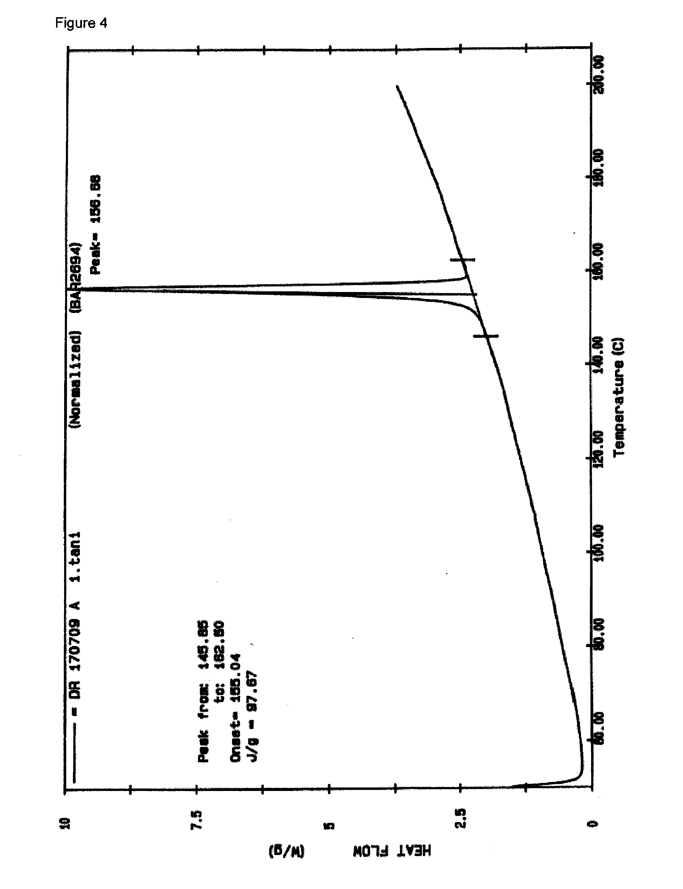

Crystalline dronedarone mesylate of the present invention has a melting temperature of 157 ± 1 °C. The DSC of this compound is shown in figure 4.

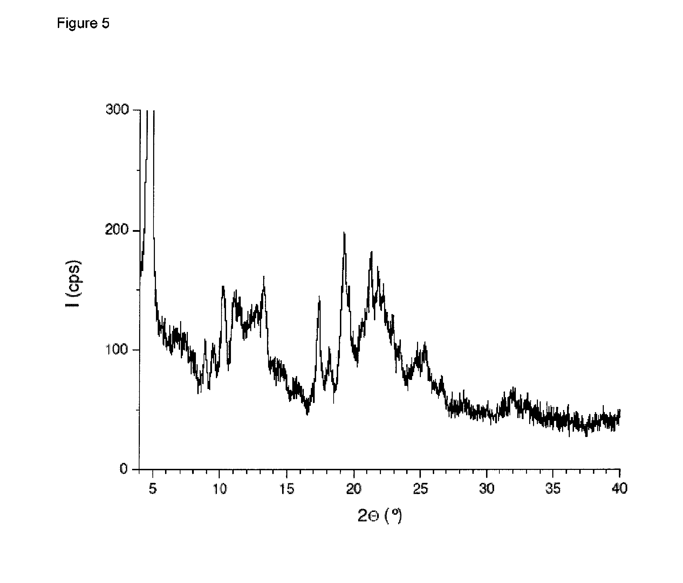

Crystalline dronedarone hemisulfate in the polymorphic form of the present invention shows an XRPD pattern having characteristic peaks at 13.3 ± 0.2, 19.3 ± 0.2 and 21.3 ± 0.2 degree 2-Theta. Preferably, the XRPD pattern has characteristic peaks at 11.0 ± 0.2, 13.3 ± 0.2, 19.3 ± 0.2, 21.3 ± 0.2 and 21.8 ± 0.2 degree 2-Theta. The XRPD pattern of crystalline dronedarone hemisulfate of the present invention is shown in figure 5. The most intense peaks of this XRPD pattern are summarized in the following table 3.

**Table 3**

| **Degree 2-Theta (± 0.2)** | **Relative Intensity** |
|---|---|
| 8.9 | 27 |
| 9.5 | 17 |
| 10.3 | 68 |
| 11.0 | 63 |
| 11.5 | 63 |
| 13.3 | 81 |
| 17.4 | 72 |
| 18.2 | 22 |
| 19.3 | 100 |
| 21.3 | 82 |
| 21.8 | 74 |
| 23.0 | 41 |
| 23.5 | 28 |
| 25.4 | 36 |
| 26.7 | 21 |

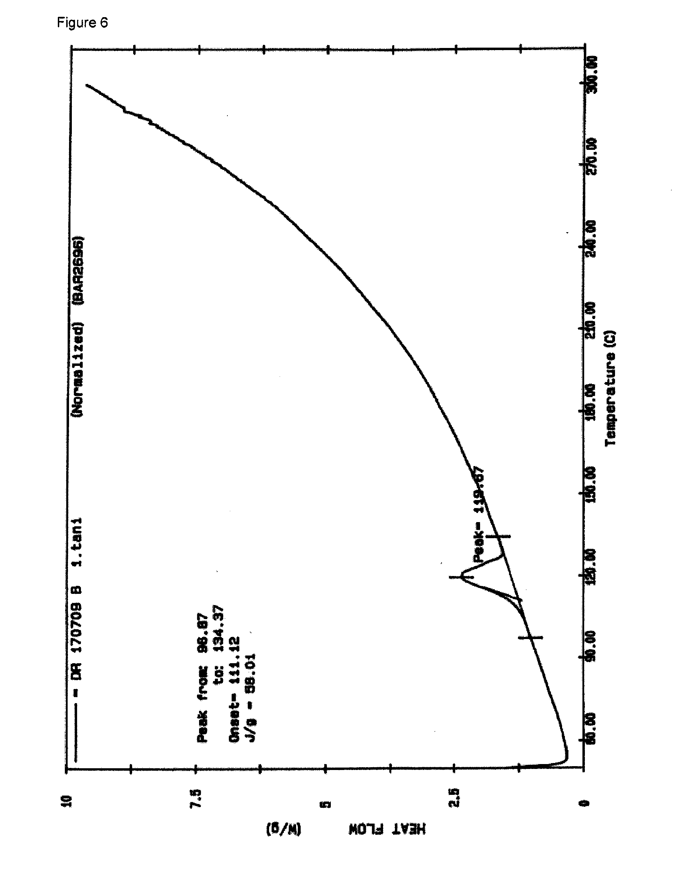

Crystalline dronedarone hemisulfate of the present invention has a melting temperature of 120 ± 1 °C. The DSC of this compound is shown in figure 6.

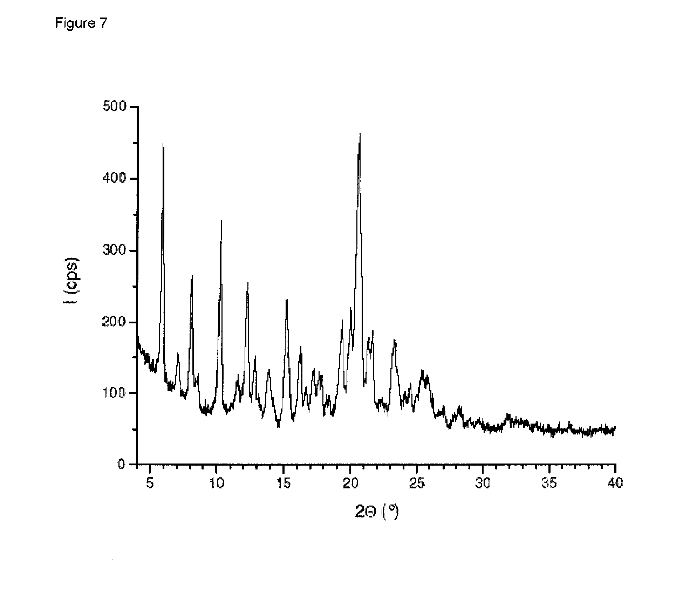

Crystalline dronedarone fumarate in the polymorphic form of the present invention shows an XRPD pattern having characteristic peaks at 6.0 ± 0.2, 10.3 ± 0.2, 15.2 ± 0.2 and 20.6 ± 0.2 degree 2-Theta. Preferably, the XRPD pattern has characteristic peaks at 6.0 ± 0.2, 8.1 ± 0.2, 10.3 ± 0.2, 12.3 ± 0.2, 15.2 ± 0.2, 20.6 ± 0.2 and 23.3 ± 0.2 degree 2-Theta. The XRPD pattern of crystalline dronedarone fumarate of the present invention is shown in figure 7. The most intense peaks of this XRPD pattern are summarized in the following table 4.

**Table 4**

| **Degree 2-Theta (± 0.2)** | **Relative Intensity** |
|---|---|
| 6.0 | 83 |
| 7.1 | 15 |
| 8.1 | 47 |
| 8.6 | 11 |
| 10.3 | 65 |
| 11.6 | 11 |
| 12.3 | 48 |
| 12.8 | 19 |
| 13.9 | 18 |
| 15.2 | 44 |
| 16.2 | 26 |
| 16.7 | 13 |
| 17.2 | 21 |
| 17.6 | 18 |
| 17.9 | 18 |
| 18.4 | 12 |
| 19.3 | 39 |
| 20.0 | 42 |
| 20.6 | 100 |
| 21.3 | 30 |
| 21.7 | 36 |
| 22.3 | 11 |
| 23.3 | 29 |
| 24.5 | 16 |
| 25.4 | 19 |
| 25.9 | 21 |

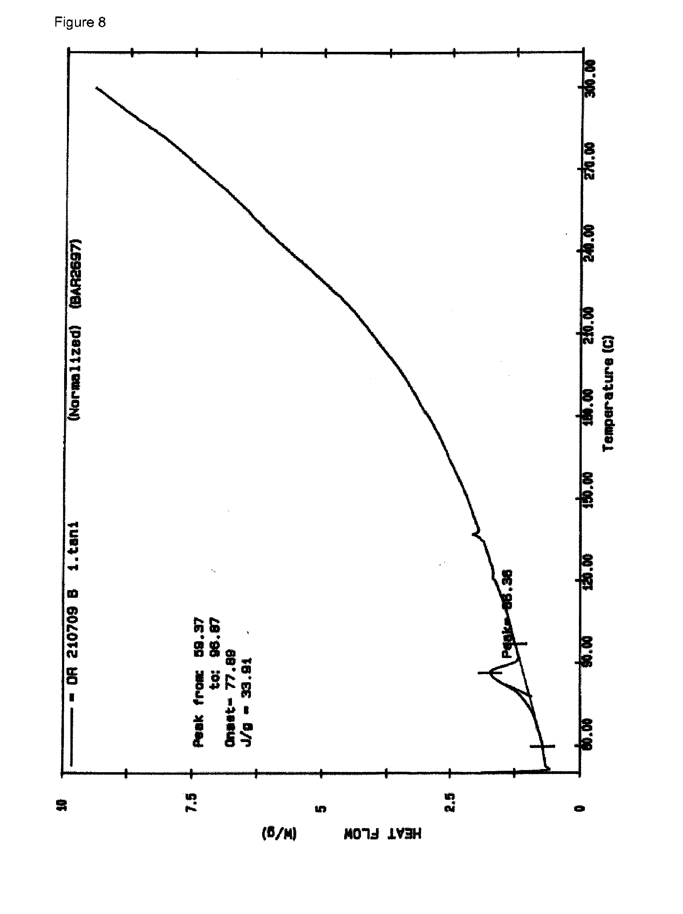

Crystalline dronedarone fumarate of the present invention has a melting temperature of 86 ± 1 °C. The DSC of this compound is shown in figure 8.

As discussed above, it was surprisingly found that the batch obtained in the synthesis of dronedarone free base contains impurities having a molecular weight being higher than the molecular weight of dronedarone free base, such as oligomers and polymers obtained as by-products in the reaction sequence. Since these impurities prevent the final product from crystallization, it is desirable to remove these impurities from the reaction product. Therefore, the present invention also relates to a process for the purification of dronedarone free base, which process comprises the step of essentially removing from a batch of dronedarone free base those impurities having a molecular weight being higher than the molecular weight of dronedarone free base.

In one embodiment of this process said impurities are removed by adding a solvent to the batch of dronedarone free base, dissolving the dronedarone free base in the solvent and removing the precipitated impurities, for example, by filtration. As solvent any solvent dissolving the dronedarone free base but not dissolving the high molecular weight impurities can be use. Suitable solvents are ethers and in particular diethyl ether. The amount of solvent is not particulary limited but it has been found that good results are obtained if the solvent is used in a high excess relative to the dronedarone free base. Suitably, the solvent is used in an amount such that the weight of the dronedarone free base relative to the weight of the solvent is in the range of 1:10 to 1:100, preferably in the range of 1:20 to 1:60, more preferably in the range of 1:30 to 1:50 or 1:40 to 1:50, such as about 1:40.

In one embodiment of the process of the present invention about one part by weight of dronedarone free base is added to about 40 parts by weight of diethyl ether and stirred for about 30 minutes at 23°C. The precipitated high molecular weight impurities are filtered off using, for example, a filter paper or sintered glass, and the filtrate containing the purified dronedarone free base is evaporated.

To additionally remove a substantial part of the remaining impurities from the dronedarone free base obtained in the above purification process, a further purification step using, for example, silica gel can be employed. For this second purification step the dronedarone free base can be dissolved in a suitable solvent, such as dichloromethane. To this solution silica gel can be added and the suspension can be stirred at room temperature for a time sufficient to remove at least part of the remaining impurities. The silica gel can then be filtered off and the filtrate can be evaporated to obtain the purified dronedarone free base.

To prepare the salts of the present invention from the dronedarone free base the purified dronedarone free base can subsequently be converted into a crystalline salt of dronedarone by mixing a solution of dronedarone free base with a suitable acid, precipitating the crystalline salt of dronedarone and collecting the precipitate, for example, by filtration. Thus, the present invention also relates to a process for the preparation of a crystalline salt of dronedarone, which process comprises any of the above-described purification processes and the subsequent preparation of the crystalline salt of dronedarone from the dronedarone free base obtained in the purification process.

Due to their crystallinity the salts of the present invention are particularly suited for the preparation of pharmaceutical compositions. Therefore, the present invention also relates to a pharmaceutical composition comprising a crystalline dronedarone salt of the present invention. Suitable pharmaceutical compositions are known to a person skilled in the art and described in the above-referenced prior art documents.

The attached figures show in
figure 1 the XRPD pattern of crystalline dronedarone hydrochloride,
figure 2 the DSC of crystalline dronedarone hydrochloride,
figure 3 the XRPD pattern of crystalline dronedarone mesylate,
figure 4 the DSC of crystalline dronedarone mesylate,
figure 5 the XRPD of crystalline dronedarone hemisulfate,
figure 6 the DSC of crystalline dronedarone hemisulfate,
figure 7 the XRPD pattern of crystalline fumarate and
figure 8 the DSC of crystalline dronedarone fumarate.

The XRPD pattern were measured under the following conditions:

| | |
|---|---|
| Instrument: | XRD-7 Seifert Company |
| Wavelength: | CuK alfa1 radiation |
| Detector: | Linear PSD |
| Monochromator: | Primary Ge (111) in the reflection mode |
| Scan Mode: | Transmittance / Moving PSD / Moving omega |
| Scan Type: | 2Theta: Omega |

The HPLC measurements were carried out under the following conditions:

| | |
|---|---|
| HPLC Apparatus: | High pressure gradient pump UV detector Column thermostat Variable volume sample injector |
| Column: | Synergi Fusion RP C18, 4.6 x 150 mm, 3.5 p (Phenomenex) |
| Pre-column: | AJO-7557 Fusion RP 4 x 3,0 mm (Phenomenex) |
| Mobile phase: | Methanol, 20 mM ammonium carbonate buffer |
| Buffer pH: | 8.5 |
| Solvent: | Methanol |
| Column temperature: | 40 °C |
| Detection: | 230 nm |
| Injection volume: | 2 µl |
| Time of analysis: | 20 minutes |

Gradient table:

| Time (min.) | % MeOH |
|---|---|
| 0.1 | 70.0 |
| 15.0 | 95.0 |
| 16.0 | 95.0 |
| 17.0 | 70.0 |
| 20.0 | 70.0 |

The retention time of dronedarone is 10.8 min.

The invention will now be explained by the following examples which are not intended to be construed as limiting.

### Examples

Dronedarone free base as used in the following examples can be prepared by any known process such as disclosed in US 7,312,345.

### Example 1 - Precipitation of impurities in ether

Base resulting from the synthesis (HPLC purity approx. 90 %) contained oligomeric impurities not detected by HPLC with UV detection that made impossible successful crystallization of any salt.

Dronedarone base (320 g) was dissolved in ether (12 L) and stirred for 30 minutes at laboratory temperature. Precipitated amorphous oligomeric impurities were filtered off through filter paper and yellowish filtrate was evaporated on Rotary Vacuum Evaporator (RVE).

Yield was 271.7 g (88 %) HPLC purity was 90%.

### Example 2 - Purification on silica gel

Dronedarone base (273.9 g) was dissolved in tenfold volume (2739 mL) of dichloromethane and the same amount of silica gel (273.9 g) was added. Suspension was stirred for one hour at laboratory temperature and then silica gel was filtered off and washed with dichloromethane (2739 mL) and mixture of dichloromethane and methanol 1:1 (2739 mL). Combined filtrates were evaporated on RVE.

Yield was 238.7 g (87 %), HPLC purity was 92 %.

### Example 3 - Precipitation of mesylate

Dronedarone base purified according to Example 1 and Example 2 (117.0 g; 0.21 mol) was dissolved in acetone and the solution was then cooled down to -20°C. Solution of methanesulphonic aicd (20.2 g; 13.6 mL; 0.21 mol) in acetone (211 mL) was added dropwise. Precipitated white suspension was stirred for one hour while the temperature was maintained bewenn -20 and -10°C. Precipitate was filtered off through sintered glass under nitrogen and washed twice with ether cooled to 0°C (500 mL). Product was dried at 40°C and 133 Pa.

Yield was 99.6 g (85 %), HPLC purity was 98 %.

### Example 4 - Preparation of dronedarone base from mesylate

Dronedarone mesylate (150 g; 0.23 mol) was dissolved in dichloromethane and the solution was neutralized with saturated solution of NaHCO₃ (approx. 100 mL). Organic layer was separated and water phase was washed with dichloromethane (150 mL). Combined organic layers were dried over anhydrous sodium sulfate and then evaporated on RVE.

Yield was 128 g (almost theoretical), HPLC purity was 97.4 %.

### Example 5 - Dronedarone hydrochloride

Dronedarone base (128.9 g; 0.23 mol) was dissolved in 2-butanone (1280 mL) and the solution was cooled down to -20°C. Hydrochloric acid (0.25 mol; 50 mL of 5M HCl in ether) was added dropwise. Precipitated white suspension was stirred for one hour at -20°C and then filtered off through sintered glass under nitrogen. Obtained crystal was washed with 2-butanone, cooled down to 0°C (200 mL) and then washed twice with ether cooled down to 0°C (500 mL).

Yield was 124.2 g (91 %), HPLC purity was 99.3 %.

### Example 6 - Recrystallization of dronedaron hydrochloride

Dronedarone hydrochloride (124.2 g; 0.21 mol) was dissolved in 2-butanone (800 mL) under reflux and then let to cool down to laboratory temperature and then cooled down to -10°C. Crystalline product was filtered off though sintered glass under nitrogen and washed with 2-butanone cooled down to 0°C (300 mL) and twice with ether cooled down to 0°C (500 mL). Product was dried at 40°C and 133 Pa.

Yield was 116.7 g (94 %), HPLC purity was 99.7 %.

Ratio of dronedarone base to hydrochloric acid was determined by argentometric titration. The determined ratio was 1:1.

### Example 7 - Dronedarone Sulfate

Dronedarone base (34.0 g; 0.06 mol) was dissolved in ether and the solution was cooled down to -20°C. Sulphuric acid (6.2 g; 0.06 mol; 3.4 mL 96-% H₂SO₄) was added dropwise. Precipitated white suspension was stirred for one hour while the temperature was maintained between -20 and -10°C. Precipitate was filtered off through sintered glass under nitrogen and washed twice with ether cooled down to 0°C (500 mL).

Yield was 21.8 g (55 %), HPLC purity was 98.5 %.

### Example 8 - Recrystallization or dronedarone sulfate

Dronedarone sulfate (21.8 g; 0.21 mol) was dissolved in 2-butanone (300 mL) under reflux and then let to cool down to laboratory temperature and then cooled down to -10°C. Crystalline product was filtered off through sintered glass under nitrogen and washed with 2-butanone cooled down to 0°C (50 mL) and twice with ether cooled down to 0°C (100 mL). Product was dried at 40°C and 133 Pa.

Yield was 10.0 g (46 %), HPLC purity was 99.8 %.

Ratio of dronedarone base to sulphuric acid was determined by alkalimetric titration. The determined ratio was 2:1 - this salt is crystallizing as hemisulfate.

### Example 9 - Dronedarone Fumarate

Dronedarone base (3.1 g; 2.6 mmol) was dissolved in acetone and the solution was cooled down to -10°C. Fumaric acid (5.6 mmol; 0.65 g in 10 mL of acetone) was added dropwise. Precipitated white suspension was ultrasonicated in ultrasonic cleaner for 10 minutes and then stirred for additional one hour at -10°C. Precipitate was filtered off through sintered glass under nitrogen and washed with acetone cooled to 0°C (10 mL) and twice with ether cooled down to 0°C (20 mL).

Yield was 2.9 g (77 %). The product contained approximately 7 % of free fumaric acid and was taken for recrystallization.

### Example 10 - Recrystallization of dronedarone fumarate

Dronedarone fumarate (2.9 g) was dissolved in acetone (10 mL) under reflux and then let to cool down to laboratory temperature and then cooled down to -10°C. Crystalline product was filtered off through sintered glass under nitrogen and washed with acetone cooled down to 0°C (10 mL) and twice with ether cooled down to 0°C (20 mL). Product was dried at 40°C and 133 Pa.

Yield was 2.1 g (72%), HPL purity was 99.8%.

Ratio of dronedarone to fumaric acid was determined by HPLC using dronedarone HCl as working standard. The determined ratio was 1:1.

### Example 11 - Recrystallization of dronedarone mesylate

Dronedarone mesylate (25.9 g) prepared according to the procedure described in Example 3 was dissolved in 2-butanone (400 mL) under reflux and then let to cool down to laboratory temperature and then cooled down to -10°C. Crystalline product was filtered off through sintered glass under nitrogen and washed with 2-butanone (100 mL) cooled down to 0°C and twice with ether (200 mL) cooled down to 0°C. Product was dried at 40°C and 133 Pa.

Yield was 21.3 g (83 %), HPLC purity was 99.8 %.

Ratio of dronedarone to methanesulphonic acid was determined by alkalimetric titration. The determined ratio was 1:1.

## Claims

1. Salt of dronedarone, which is crystalline.

2. Salt of dronedarone according to claim 1, which has a chemical purity of > 99 %.

3. Salt of dronedarone according to claim 1 or 2, which is substantially free of impurities having a molecular weight being higher than the molecular weight of dronedarone free base.

4. Salt of dronedarone according to any of the preceding claims, which is selected from the group consisting of dronedarone hydrochloride, dronedarone mesylate, dronedarone hemisulfate and dronedarone fumarate.

5. Crystalline dronedarone hydrochloride, showing an XRPD pattern having characteristic peaks at 7.7 ± 0.2, 13.8 ± 0.2, 21.7 ± 0.2 and 26.2 ± 0.2 degree 2-Theta.

6. Crystalline dronedarone hydrochloride according to claim 5, showing an XRPD pattern essentially as in figure 1.

7. Crystalline dronedarone mesylate, showing an XRPD pattern having characteristic peaks at 7.2 ± 0.2, 10.6 ± 0.2, 20.3 ± 0.2 and 22.3 ± 0.2 degree 2-Theta.

8. Crystalline dronedarone mesylate according to claim 7, showing an XRPD pattern essentially as in figure 3.

9. Crystalline dronedarone hemisulfate, showing an XRPD pattern having characteristic peaks at 13.3 ± 0.2, 19.3 ± 0.2 and 21.3 ± 0.2 degree 2-Theta.

10. Crystalline dronedarone hemisulfate according to claim 9, showing an XRPD pattern essentially as in figure 5.

11. Crystalline dronedarone fumarate, showing an XRPD pattern having characteristic peaks at 6.0 ± 0.2, 10.3 ± 0.2, 15.2 ± 0.2 and 20.6 ± 0.2 degree 2-Theta.

12. Crystalline dronedarone fumarate according to claim 11, showing an XRPD pattern essentially as in figure 7.

13. Process for the purification of dronedarone free base, which process comprises the step of essentially removing from a batch of dronedarone free base those impurities having a molecular weight being higher than the molecular weight of dronedarone free base.

14. Process according to claim 13, wherein the impurities having a molecular weight being higher than the molecular weight of dronedarone free base are removed by adding a solvent to the batch of dronedarone free base, dissolving the dronedarone free base in the solvent and removing the precipitated impurities.

15. Process for the preparation of a crystalline salt of dronedarone, which process comprises the purification process of claim 13 or 14 and the subsequent preparation of the crystalline salt of dronedarone from the dronedarone free base obtained in the purification process.

16. Pharmaceutical composition comprising a crystalline dronedarone salt according to any of claims 1-12.
